(19) Europäisches Patentamt
European Patent Office
Office européen des brevets

(11) Publication number: **0 398 817 B1**

# (12) EUROPEAN PATENT SPECIFICATION

(45) Date of publication of patent specification: **26.07.95**

(51) Int. Cl.$^6$: **C12P 21/00**, A61K 38/20

(21) Application number: **90401343.0**

(22) Date of filing: **21.05.90**

The file contains technical information submitted after the application was filed and not included in this specification

## (54) Interleukin 1 inhibitor.

(30) Priority: **19.05.89 US 354330**
**12.04.90 US 508999**
**17.05.90 US 525274**

(43) Date of publication of application:
**22.11.90 Bulletin 90/47**

(45) Publication of the grant of the patent:
**26.07.95 Bulletin 95/30**

(84) Designated Contracting States:
**AT BE CH DE DK ES FR GB GR IT LI LU NL SE**

(56) References cited:

**LYMPHOKINE RESEARCH, vol. 7, no. 3, 1988, New York, NY (US); V. BARAK et al., no. 1.32&NUM;**

**LYMPHOKINE RESEARCH, vol. 6, no. 1, 1987, New York, NY (US); V. BARAK et al., no. 1133&NUM;**

**EUROPEAN JOURNAL OF IMMUNOLOGY, vol. 16, 1986, VCH VerlagsgesmbH, Weinheim (DE); V. BARAK et al., pp. 1449-1452&NUM;**

(73) Proprietor: **HADASSAH MEDICAL ORGANIZATION**
**Kiryat Hadassah**
**P.O. Box 12000**
**IL-91120 Jerusalem (IL)**

(72) Inventor: **Treves, Avi**
**Bar-Kochva 57**
**Jerusalem (IL)**
Inventor: **Barak, Vivian**
**Hertzel Street 103**
**Jerusalem (IL)**

(74) Representative: **Warcoin, Jacques et al**
**Cabinet Régimbeau,**
**26, avenue Kléber**
**F-75116 Paris (FR)**

**EP 0 398 817 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present application is a continuation-in-part of Application Serial No. 07/508,999, filed April 12, 1990 which in turn is a continuation of application Serial No. 07/354,330, filed May 19, 1989, each of which is incorporated by reference in its entirety.

### 1. INTRODUCTION

The present invention concerns a substantially pure protein that inhibits the activity of interleukin 1 (IL-1). In vitro, this approximately 52 kD protein binds to the IL-1 cell-surface receptor and inhibits the effect of IL-1 in the comitogenic assay of moue thymocytes. In vivo, it inhibits the induction of IL-1 of fever, leukocytosis and local lymph node enlargement in mammals.

The inhibitor is secreted by a human, myelomonocytic leukemia-derived cell line. Media conditioned by these cells is purified by ion-exchange chromatography, ion-exchange HPLC and gel-filtration HPLC to isolate the inhibitor. Besides utility as an anti-inflammatory agent, the inhibitor could be useful in the treatment of cancer and autoimmune diseases. Other uses for the inhibitor may be found as an immunosuppressant in areas such as organ tranplantation: pre-treatment with the inhibitor of both the donor tissue and the receptive host could avoid rejection of the tissue and/or graft-versus-host disease.

### 2. BACKGROUND OF THE INVENTION

Interleukin 1 (IL-1) is one of the key mediators of the body's response to microbial invasion, inflammation, immunological reactions and tissue injury. Although the macrophage is a primary source of IL-1, it is also secreted by other cells, such as synovial fibroblasts, keratinocytes and Langerhans cells of the skin; mesangial cells of the kidney; B lymphocytes, natural killer cells, astrocytes and microligial cells of the brain; vascular endothelial and smooth muscle cells; corneal, gingival and thymic epithelial cells; and some T lymphocyte cell lines. Nonetheless, the monocyte remains an important source of IL-1 because of its strategic location and its ability to synthesize large amounts of IL-1 and process the IL-1 precursor more effectively than other cells.

IL-1 also acts like a hormone and induces a broad spectrum of systemic changes in neurological, metabolic, hematologic and endocrinologic systems. IL-1 also affects both destructive and repair processes during mesenchymal tissue changes. One of the major biological activities of IL-1 is induction of maturation and differentiation in T lymphocytes, and this activity is associated with IL-1 activity in T-cell mediated immunity.

The role of IL-1 in inflammation is mediated by stimulation of the production of arachidonic acid metabolites. IL-1 also acts synergistically with other cytokines, particularly tumor necrosis factor (TNF). In fact, IL-1 and TNF share several biological properties and the combined effects of these two distinct cytokines is often greater than either one alone. IL-1 also potentiates the responses of interleukin 2 (IL-2) and the interferons. IL-1 can be found in serum, culture supernatants and urine in a predominant size of 17 kD, with higher 30 kD and 64 kD and lower 2-4 kD forms also occurring. Two forms of IL-1, $\alpha$ (pI = 5) and $\beta$ (pI = 7), have been isolated. The genes that encode for both forms have been cloned from mice and humans, and both encode a 31 kD inactive precursor protein which is cleaved to smaller biologically active forms. Membrane bound IL-1 (22 kD) seems to be biologically active and mainly of the $\alpha$ form, while IL-1 released from cells is mainly $\beta$ form.

IL-1 production in vitro is augmented by a large variety of inert, immunological and microbial agents. Lipopolysaccharide (LPS) is the most potent stimulator of IL-1 production by human macrophages. Macrophages appear to possess the ability to produce IL-1 at all stages of their differentiation.

Receptors for IL-1 are found on several different types of cells. There is evidence for high affinity receptors, which undergo internalization and therefore down-regulate IL-1, as well as low affinity receptors that down-regulate IL-1 to a lesser extent.

The large amount of data available on the functions of IL-1 presents an array of vast complexity, while much less is known regarding the regulation of IL-1 activities. Only corticosteroids block IL-1 transcription and reduce its production. Induction of adrenocorticotrophic hormone (ACTH) by IL-1 may also be a negative feedback loop. However, the body may produce its own substances that inhibit the biological activity of IL-1 by binding to the molecule, by interfering with the IL-1 receptor, or by suppressing the effect of IL-1 on a variety of cells. Several factors have been described which specifically inhibit IL-1 activity. These factors were derived from different sources and differ in their biochemical structure.

A number of laboratories have described the inhibition of the thymocyte proliferation-inducing effects of IL-1 by factors that are present in serum, urine and the supernatant of cultured cells. Dinarello et al. (J. Immunol. 127:2517, 1981) described such a factor that was present in the serum of humans 3-4 hours after the administration of endotoxin. It was nondialyzable and was destroyed at 70 °C but retained activity at 56 °C. Moreover, the factor bound to, and could be eluted from, an immobilized anti-IL-1 antibody, suggesting either that the it was structurally related to IL-1 or that it bound to IL-1.

Culture supernatants from a number of different cell types also have been found to yield inhibitors of IL-1. Amento et al. (pNAS 79:5307, 1982) reported that a human macrophage-like cell line, U937, might produce an inhibitor of IL-1. Scala et al. (J. Exp. Med. 159:1637, 1984) have described an inhibitor of IL-1 that is produced by an Epstein-Barr virus-transformed B-cell line, ROHA-9. This inhibitor seems to be specific for IL-1, since it did not block the effects of another T-cell growth factor and could be absorbed by thymocytes. Wilkins et al. (Cell. Immunol. 75 :328, 1983) described an inhibitor that was present in the supernatant of U937 cells that were stimulated with concanavalin A (Con A). However, this factor inhibited mitogen-induced peripheral blood lymphocyte proliferation, and was not directly tested for IL-1 inhibitory activity. It had an apparent molecular mass of 65 kD and was partially inactivated at 56 °C. It was not cytotoxic and its effects were reversible even after 24 hours of incubation with lymphocytes. Fujiwara and Ellner (J. Immunol. 136:181, 1986) showed that U937 cells spontaneously produce an 85 kD suppressor factor that inhibits both IL-1 and IL-2 induced proliferation of thymocytes. A similar activity has been found in the supernatants of cultured human mononuclear cells by Shou et al. (Proc. Nat. Acad. Sci (USA) 77:6096, 1980). The relationship between the U937, ROHA-9, and this normal mononuclear cell-derived factor remains to be determined.

Several laboratories have also described IL-1 inhibitory factors in urine. Liao et al. (J. Exp. Med. 59 :126, 1984) have noted that the urine of febrile patients contains elevated amounts of a material that inhibits IL-1 induced thymocyte proliferation. This material is apparently specific for IL-1, as it does not block IL-2 induced thymocyte proliferation and is not cytotoxic for these cells.

Kimball et al. (J. Immunol. 133:256, 1984) described a similar inhibitor in human urine, but noted that this factor did not block another IL-1 induced effect, the induction of fibroblast proliferation. Balavoine et al. (J. Clin. Invest. 78:1120, 1986) described a 25-35 kD inhibitor of 455Il-1 induced thymocyte proliferation that was present in the urine of patients with acute monocytic leukemia. In vitro, this inhibitor also blocked IL-1 induced prostaglandin and collagenase production in fibroblasts.

Finally, Muchmore and Decker (Science 229:479, 1985) have recently described an 85 kD glycoprotein isolated from human urine during pregnancy (uromodulin) that blocks tetanus toxoid-induced human lymphocyte proliferation in vitro. Brown et al. (Proc. Nat. Acad. Sci. (USA) 83:9119, 1986) have recently shown that uromodulin is an inhibitor of IL-1 induced thymocyte proliferation. It is also distinct in its biological functions compared to the febrile inhibitor: uromodulin enhances IL-2 induced proliferation. In addition, Muchmore and Decker, id. noted that human urine contains other immunosuppressive factors, some of which bind to Con A and co-chromatograph with urine proteins on Sephadex G-75. At present, there is no information on the relationship between the urine IL-1 inhibitors and those found in serum and in culture supernatants.

Other inhibitors from zymosan-stimulated normal macrophages and mononuclear cells were described by Tiku et al. (J. Immunol. 136:3686, 1986). There were two peaks of inhibitory activity: 45-70 kD and greater than 160 kD.

Human monocytes, stimulated with immune complexes, Were found by Arend, et al. (J. Immunol. 134:3868, 1985) to secrete a 22 kD inhibitor of IL-1. Monocytes infected with respiratory syncytial virus or influenza virus secreted both IL-1 and a 45-60 kD inhibitor of IL-1, as described by Roberts et al. (in Progress in Leukocyte Biology vol. 2, pp. 409-18, Roberts et al., ed. 1985).

AIDS patients were also found by Berman et al. (Immunopathol. 42:133, 1987) to secrete an inhibitor of IL-1, with a molecular weight of 50-100 kD, that masks the high production of IL-1.

Barak et al. (Eur. J. Immunol. 16:1449, 1986 Lymphokine Res. 6(1), 1987, Abstr. N° 1133) reported the isolation of a factor secreted by a myelomonocytic cell line M20 which specifically inhibited IL-1 activity in vitro. The factor was found to be protein of 52 kD and was isolated as a single peak in ion-exchange chromatography.

## 3. SUMMARY OF THE INVENTION

The present invention is directed to a substantially pure, approximately 52 kD protein that inhibits the activity of IL-l. The invention also provides a continuous cell line that produces the protein, and methods for its purification. The invention is also directed towards the use of the inhibitory protein, both in vitro and in vivo, in reducing the proliferation of leukocytes and inflammation as well as uses in immunosuppression and the treatment of cancer.

## 4. BRIEF DESCRIPTION OF THE FIGURES

Figure 1: fractions from a DEAE ion-exchange chromatography (0.0-0.6 M NaCl gradient) of supernatant from M20-2 cells were tested for IL-1 inhibition in the mouse thymocyte co-mitogenicity assay. Data points represent the CPM of incorporated thymidine for quadruple cultures, and the horizontal line indicates the activity in control cultures with IL-1 alone, in the absence of the inhibitor.

Figure 2: fractions from an ion-exchange HPLC (0-1 M NaCl gradient) were tested for IL-1 inhibition in the mouse thymocyte co-mitogenicity assay. Data points represent the CPM of incorporated thymidine for triplicate cultures, with the horizontal line indicating the activity in cultures with IL-1 alone.

Figure 3: fractions from an ion-exchange FPLC, tested for IL-1 inhibition in the co-mitogenicity assay as before.

Figure 4: fractions number 10 and 18 from the comitogenicity assay of ion-exchange FPLC fractions indicated in Figure 3 were titrated, to determine their change in inhibitory activity with decreasing concentration by the fraction 10. Closed co-mitogenicity assay. Open squares: diamonds: fraction 18.

Figure 5: fractions from an ion-exchange chromatography were subjected to gel-fitration HPLC. The resulting fractions were tested for IL-1 inhibition in the co-mitogenicity assay, and the peak of inhibitory activity was indicated at a molecular weight of approximately 50 kD. Cross hatching: peak of inhibitor.

Figure 6: SDS-PAGE of IL-1 inhibitor. Lane 1 molecular weight markers (BSA, 67 kD; ovalbumin, 45kD; pepsin, 34.7kD; trypsinogen, 24kD; ysozyme, 14.3dD). Lane 2: peak of inhibitory activity, based on the co-mitogenicity assay, from a DEAE ion-exchange chromatography. Lane 3: peak of inhibitory activity from a subsequent gel-filtration chromatography, using the material from the prior ion-exchange chromatography.

Figure 7: the relationship of heat-treatment IL-1 inhibitor and retention of its ability to bind to the IL-1 receptor was determined by an in vitro binding assay. Open squares: inhibitor exposed to 56 °C for 20 minutes. Closed circles: inhibitor exposed to 100 °C for 2 minutes. Open circles: control inhibitor (no heat treatment).

Figure 8: a comparison, by the in vitro binding assay, between IL-1 and the IL-1 inhibitor and their ability to disassociate from the IL-1 receptor. Open circles: pre-incubation of the inhibitor. Crosses: no pre-incubation.

Figure 9: IL-1 and various amounts of the inhibitor were injected concomitantly into mice, and the percent-inhibition of fever was determined at different time points. Open squares: inhibition 0.5 hours after concomitant injection. Crosses: 1 hour after injection. Diamonds: 1.5 hours after injection. Triangles: 2 hours after injection.

Figure 10: IL-1 was injected into mice from 0 to 24 hours after the inhibitor, and the percent-inhibition of fever was determined at two different time points. Cross-hatching to the upper-right: inhibition 1.5 hours after IL-1 injection. Cross-hatching to the upper-left: inhibition I hour after IL-1 injection.

Figure 11: various amounts of IL-1, with and without the inhibitor, were injected into mice, and the percent-inhibition of leukocytosis induction was determined at different time points. Each line of experiments represents the determination of the index of leukocytosis at 1, 2, 3 or 4 hours, respectively, after the injection. Open bars: IL-1 alone. Cross-hatched bars: IL-1 plus 2 units of inhibitor.

Figure 12: IL-1 was injected into mice from 0 to 24 hours after the inhibitor, and the percent-inhibition of leukocytosis was determined at two different time points. Cross-hatching to the uper-right: inhibition 2 hours after IL-1 injection. Cross-hatching to the upper-left: inhibition 3 hours after IL-1 injection.

## 5. DESCRIPTION OF THE INVENTION

The present protein is an.IL-I inhibitor which exhibits a number of clinically useful properties which have not previously been described in the art. The protein in question has a molecular weight of 52 ± 4 kD determined by means of gel filtration and SDS PAGE, with an isoelectric point of 4.1 ± 0.2. The protein is obtainable by ion-exchange chromatography and HPLC purification from the supernatant of a myelomonocytic cell line designated M20-2. Although an IL-1 inhibitory protein from the cell line M20 has been previously described (Barak et al., Eur. J. Immunol. 16:1449-1452, 1986 Lymphokine Res. 6(1), 1987, Abstr. N° 1133), the present IL-1 inhibitor, derived from a subline of M20, has a higher level of activity than has been found in the protein from M20.

As outlined below, the claimed protein has a high level of anti-inflammatory activity. Three art-recognized tests are typically employed to determine a given compound's ability to affect the inflammatory response, namely: effect on fever, leukocytes and enlargement of lymph glands. In each of these tests, the present inhibitor had a significant effect in vivo in reducing each of these characteristic inflammatory responses. These results indicate a significant therapeutic potential for the novel inhibitor.

It is to be understood that the identity of the IL-1 inhibitor is not so limited and encompasses various other amino acid substitutions, additions or deletions to the amino sequence of the described IL-1 inhibitor.

Another embodiment of the present invention is recombinant production of the described IL-1 inhibitor. Such process will employ DNA sequences that code for the IL-1 inhibitor, and includes transforming with the recombinant DNA molecules, characterized by those sequences, various unicellular hosts to produce the IL-1 inhibitor or portions thereof by fermentation of the transformed host. The present invention also employs the described IL-1 inhibitor to determine the molecular structure and location of the active sites of the IL-1 inhibitor and uses that information to design fragments and peptides for use as a IL-1 inhibitor and for recombinant production as described in the uses and methods of the present invention.

The following is an outline of the techniques involved in the invention.

### 5.1. METHOD OF PURIFICATION

A number of methods have been found useful in purifying the subject IL-1 inhibitor from supernatant of the M20-2 cell line. Briefly, a satisfactory level of purity, i.e., 1 units of activity/3 µg of protein, has been obtained by ion-exchange chromatography, followed by further purification or ion exchange HPLC or FPLC. Alternately, ion exchange HPLC alone will provide a substantially pure product. In a preferred embodiment, the inhibitory protein is purified by isoelectric focusing. A brief description of each procedure is outlined below, and in greater detail in the examples in Section 6.

While the protocols noted above were used for each step of the purification, it is within the skill of the art to alter them slightly without significantly changing the results.

#### 5.1.1. ION-EXCHANGE CHROMATOGRAPHY

Supernatant from M20-2 cells, containing the IL-1 inhibitor, was subjected to ion-exchange chromatography as noted above, and the resulting fractions were tested for inhibition in the co-mitogenicity assay. Fractions 11-15 (11-15), the peak of inhibitor activity (Fig. 1), were pooled, lyophilized and frozen for further characterization.

#### 5.1.2. ION-EXCHANGE HPLC

The pooled fractions, with peak IL-1 inhibitor activity, from the ion-exchange chromatography were further purified by ion-exchange HPLC.

Fractions 11-15 from the ion-exchange chromatography were subjected to both HPLC and FPLC. In the former, 300 mg of the lyophilized IL-1 inhibitor was reconstituted in 2.0 ml of water and loaded onto a TSK DEAE-5PW HPLC column (Biorad) with a 0.0 to 0.1 M NaCl gradient in 20mM Tris (pH 7.5) for 1 ml fractions per minute. Figure 2 shows the inhibitory activity of the fractions, in pooled sets of two consecutive aliquots, as determined by a slightly modified co-mitogenicity assay. In the latter, 1.0 mg of the same inhibitor was reconstituted in 100 ml of water and loaded onto a Mono Q HR 5/5 FPLC column (Pharmacia) under the same conditions (except pH 8.0). The inhibitory activity of the fractions was determined as noted above (Fig. 3).

The IL-1 inhibitor activity from the FPLC was titrated, using the co-mitogenicity assay under the conditions noted above (Fig. 4). Increasing amounts of both fractions 10 and 18 showed a similar increase in the inhibition of the co-mitogenic effect of IL-1.

#### 5.1.3. GEL-FILTRATION HPLC

Lyophilized material from an ion-exchange chromatography was run on a Sorbax GF 250 HPLC column (Dupont). In Figure 5, the peak of IL-1 inhibitor activity (based on the co-mitogenicity assay noted above) is indicated at a molecular weight slightly less than that for the BSA standard, 68 kD, suggesting that in an unreduced state the inhibitor is approximately 50 kD. In this instance, the lower inhibitory activity is explained by the presence of M20-2 derived growth factors of similar size. Because this factor can not substitute for either IL-1 or IL-2 when assayed, it is apparently another type of growth factor.

### 5.1.4. ISOELECTRIC FOCUSING

Crude supernatant from M20-2 cells were also directly subjected to isoelectric focusing, without any prior purification. Of the 20 fractions produced, the peak of IL-1 inhibitor activity, as determined by the comitogenicity assay, was consistently found in the ninth fraction, which corresponds to a pI point of 4.1. The fraction was found to contain 300 units of activity, or 1 unit per 3 mg of protein. This fraction produced a single band of approximately 52 kd on a non-denaturing, 10% gel stained with coomassie blue; when slices of this gel corresponding to a range of molecular weights were sectioned and individually tested in the co-mitogenicity assay, the band produced by the ninth fraction corresponded exactly with IL-1 inhibitor activity. There was no inhibitory activity in any of the other slices of the gel corresponding to other molecular weights, except for the very low molecular weight area at the bottom of the gel. Because activity was found in this region even for control runs that did not contain any fractions from the isoelectric focusing, it was not due to degradation of the IL-1 inhibitor. This phenomenon is possibly due to break-down products of the gel itself.

### 5.2. BIOCHEMICAL CHARACTERIZATION

The present inhibitor has been shown to be effective, in co-mitogenicity assays of inhibiting the co-mitogenic effect of IL-1 on mouse thymocytes. In IL-1 receptor binding assays, the inhibitor has also been shown to be capable of blocking the binding of IL-1 $\alpha$ and $\beta$ to the receptor; this inhibitory activity is reduced, however, by treatment of the inhibitor at 100°C for a short period of time, indicating the proteinaceous nature of the inhibitor. The binding of the inhibitor to the IL-1 receptor site is also capable of reversal, as is the binding of IL-1 itself.

Comparison with similar inhibitors, e.g., the inhibitor derived from cell line M20, shows that the present inhibitor is substantially more active than the inhibitor from M20 when compared in comitogenicity assays.

### 5.3. ACTIVITY IN VIVO

For all of the in vivo assays, recombinant human IL-1 (Cistron) and recombinant mouse IL-1 (Dr. Ivan Otterness, Pfizer) were dissolved in PBS (pH 7.2) and stock solutions of 10 units/ml were stored at -20°C. "One unit of activity" for IL-1 was defined as the amount required to augment by 50% the response of mouse thymocytes to PHA in the comitogenicity assay. In addition, IL-1 inhibitor purified by ion-exchange chromatography (gradient 0.0 to 0.4 M NaCl) was used in all of the in vivo assays. Fractions were dialyzed against RPMI 1640 and stored at -20°C, with "one unit of activity" for the inhibitor of IL-1 defined as the amount required to inhibit by 50% the co-mitogenic activity of one unit of IL-1. For both IL-1 and its inhibitor, as well as Con A and LPS, titration of activity was performed at twofold dilutions in order to determine the number of units used. The percentage of inhibition was calculated as:

$$100 \times \left(1 - \frac{\text{ACTIVITY WITH INHIBITOR}}{\text{ACTIVITY OF IL-1 ALONE}}\right)$$

In all of the in vivo experiments, male Balb/c mice, 6 to 8 weeks old, were injected with PBS solutions of Con A, LPS or IL-1, alone or concomitantly with the IL-1 inhibitor, and each experimental group usually consisted of four mice.

The experiments to assay the inhibition of fever induction were started only after there had been 3 consecutive and similar baseline temperature readings in each mouse. Rectal temperature was measured with a No. 402 Thermistor Probe (Yellow Springs Instruments, Yellow Springs, Ohio), inserted to a distance of 2 cm for about 30 seconds and recorded on a Yellow Springs Telethermometer. Mice were gently restrained during the procedure by their confinement in a size-compatible, ventilated tube. Experiments were performed in which either 0.25, 0.5, 1.0, 1.5 or 2.0 units of IL-1 inhibitor and 150 units human IL-1 were intravenously injected concomitantly, and in which 2 units of IL-1 inhibitor was intravenously injected from 2 to 24 hours before the 150 units of IL-1.

To measure the inhibition of leukocytosis induction, blood samples were taken from the mice by venous puncture of the tail and stored in heparinized tubes, with total leukocyte counts determined by a Coulter Model S-Plus (Coulter Electronics, Hialeah, Florida). In one experiment, either 50, 100, 150 or 200 units of human IL-1 were intravenously injected either alone or concomitantly with 2 units of inhibitor; in another, 2 units of inhibitor were intravenously injected from 2 to 24 hours before 150 units of IL-1.

To assay the inhibition of lymph node-enlargement, 0.05 ml of the sample to be tested was injected subcutaneously into both right foot pads of a mouse to cause a response in the local draining lymph node, which was measured by an increase in lymph node weight. After 3 days, the mice were anesthetized with ether and sacrificed by cervical dislocation. The popliteal lymph nodes from both the right and left sides were removed, trimmed of fat and weighed. After previously determining the dose response and kinetics for optimal lymph node enlargement, those amounts, 12.5 µg Con A, 50 µg LPS, 125 units mouse IL-1 and 100 units human IL-1, were injected either individually or in combination with 2 units of IL-1 inhibitor. Also, 1 unit of IL-1 inhibitor was injected either from 2 hours to 4 days before 125 units of mouse IL-1 or from 2 hours to 1 day after the IL-1.

## 5.4. THERAPEUTIC USE OF IL-1 INHIBITOR

The data presented below show the utility of the instant inhibitor as an anti-inflammatory agent, presumably as a result of its inhibitory effect on IL-1. Thus, the present invention also provides a method for prevention or treatment of an undesired inflammatory response. Of particular interest are those diseases in which an imbalance between IL-1 and its natural inhibitors cause the disease state. Among conditions which would involve undesirable inflammatory responses are allergic reactions, chronic inflammatory diseases, autoimmune diseases (e.g. rheumatoid arthritis) and high fevers associated with cancer or infectious conditions. The active component is preferably formulated into a composition in combination with a pharmaceutically acceptable carrier. Administration of the composition may be either oral or by local or parenteral injection. An effective unit dose form of the present composition would comprise approximately 0.0001-1.0 µg of the active component per kg of body weight. However, it is within the skill of the experienced physician to adjust the dose in accordance with the patient's needs and the condition to be treated.

## 5.5. OTHER USES

The claimed inhibitor also has significant non-therapeutic uses as a tool in identification of the gene sequence. It is within the scope of the invention to produce large amounts of the IL-1 inhibitor, substantially purified from other human proteins, using the DNA sequences, along with included DNA molecules in unicellular hosts transformed with the DNA sequence. The purified IL-1 inhibitor can be used as a source of amino acid sequence data for use in designing DNA probes which can be used to isolate and select DNA sequences coding for the IL-1 of this invention.

In particular, the amino acid sequence of various sites and fragments of the purified IL-1 can be determined and used to deduce the DNA sequences coding for them to design DNA probes which are potentially useful to screen various DNA libraries for DNA sequences coding for the IL-1 inhibitor. Sources of such libraries can include chromosomal gene banks and DNA or cDNA libraries of tissue or all cell lines producing the IL-1 inhibitor of the invention. Methods for preparing and cloning cDNA and expressing an IL-1. inhibitor, screening of a library of clones for an IL-1 insert and the use of the DNA sequences expression in a host/vector combination are described in PCT International Application, Publication Number WO 89/01946, which is incorporated by reference herein in its entirety and are considered within the scope of the present invention.

Briefly, preparing cDNA involves isolating poly $A^{+}$mRNA from an IL-1 inhibitor producing cell source, followed by construction of a cDNA library from the isolated poly $A^{+}$mRNA. The library of clones are then screened for the recombinant DNA molecule containing the IL-1 inhibitor. The utilizing various approaches. One approach may include the use of DNA probes to screen cDNA or genomic libraries that encode for IL-1 inhibitor. The DNA probes consist of a series of synthetic DNA fragments which are constructed based on partial amino acid sequences of the purified IL-1 inhibitor determined by techniques well known in the art. The selected sequences may then be used to transform and express the IL-1 inhibitor of the present invention in prokaryotic and eukaryotic hosts. The DNA sequences of the present invention may also be utilized in vectors consisting of segments of chromosomal, non-chromosomal and synthetic DNA and expressed in a variety of host/vector combinations to produce IL-1 inhibitor. Derivatives of SV40 and known bacterial plasmids as expression vectors with various sites of inserted IL-1 inhibitor DNA sequences and a control sequence, and other vectors capable of expression in prokaryotic and eukaryotic hosts can be employed. The present invention thus encompasses the 52 KD protein or its equivalents, made by any means, and is not merely restricted to a protein which has been isolated from the M20-2.

## 6. EXAMPLE: PURIFICATION AND ACTIVITY OF THE INHIBITOR

The following is an example of the purification process, characterization and activity assays for the IL-1 inhibitor from M20-2 cells.

### 6.1. METHOD OF PURIFICATION

At each stage of puri.fication, IL-1 inhibitor activity was measured by the inhibition of the IL-1 augmentation of the mitogenic response in mouse thymus cells to PHA, as described (Barak et al., Eur. J. Immunol. 16: 1449-1452, 1986). The inhibition of this IL-1 comitogenic activity was indicated by comparing the reduction in $^3$(H)-thymidine incorporation in the growing cells, in the presence of the inhibitor, with the incorporation in the growing cells in the presence of IL-1 alone. C3H/HeJ mouse thymocytes were cultured for 48 hours in 96 well plates, containing 1.0 x $10^6$ cells, 1 unit of recombinant human IL-1 (Cistron Bioteohnology, Pine Brook, NJ) and 500 ng of phytohemagglutinin (PHA) in 150 µl of RPMI 1640 culture medium (with 10% fetal calf serum and $5x10^{-5}$ M β-mercaptoethanol) per well. The PHA mitogen concentration was previously determined by titration in the thymocyte assay. Cultures were pulse-labeled with 1.0 µC of 3(H)-thymidine before harvesting 24 hours later and data were based on counts per minute (CPM) of triplicate or quadruplicate cultures.

For the first step of purification by ion-exchange chromatography, 100 ml of supernatant from the cultured media of myelomonocyte cell line M20-2, a subline of M20 (Barak et al., Eur. J. Immunol. 16:1449-1452, 1986 Barak et al., Lymphokine Res. 6(1), 1987, Abstr. N° 1133.), was applied to a column (2.5 x 20cm) of DEAE Sephacel (Pharmacia, Uppsala, Sweden), as described in Barak et al., id. The column was eluted with 50 mM Tris HCl buffer, pH 7.4 (200 ml), and then developed with a linear gradient of NaCl (0.0 to 0.6 M). Fractions were collected, dialyzed against RPMI 1640, filtrated and tested in 20, 50 and 100 µl aliquots per well for the inhibition of IL-1 activity in the co-mitogenicity assay of mouse thymocytes with PHA.

In the next step of purification by ion-exchange HPLC, initially lyophilized protein from the ion-exchange chromatography, after being run on a PD10 "desalting column" (G-10 column from Pharmacia), was reconstituted in water and run on an HPLC or FPLC column. From each resulting fraction, the IL-1 inhibitory activity of 10 µl aliquots was determined in the co-mitogenicity assay by measuring the reduction of 3(H)-thymidine incorporation in the proliferating thymocytes.

As an alternative process for purifying the IL-1 inhibitor, supernatant from the cultured media of M20-2 cells can be directly run on an ion-exchange HPLC column, without any prior purification.

In order to further characterize the IL-1 inhibitor lyophilized material from the ion-exchange chromatography was subjected to gel-filtration HPLC with a Sorbax GF 250 column (Dupont). Phosphate buffered saline (PBS, pH 7.4) was used as the buffer and 25 mg of the material was reconstituted in 250 µl of PBS, loaded as the sample and run at 0.5 ml per minute. From each 0.5 ml fraction, a 30 µl aliquot was analyzed for IL-1 inhibitory activity, as before, in the co-mitogenicity assay.

In a preferred embodiment of the invention, the Rotofor cell (Bio-Rad, Richmond, CA) was used, which is a new development in preparative isoelectric focusing, based on the prototype described by Egen et al. (in Electrophoresis '83, H. Hirai ed., Walter de Gruyter & Co., Berlin, New York, pp. 547-550 (1984). Proteins were separated in this method by isoelectric focusing in free solution. The starting material for the isolation of the inhibitor was 1 liter of crude supernatant obtained from the cell line M20-2. The supernatant was lyophilized and the powder obtained (15 g) was dissolved in RPMI 1640 and dialyzed against 1000 volumes of RPMI 1640 diluted 1:150. Prior to the run, the sample, containing 1.25-1.85 mg/ml of protein, was diluted 20-fold with 10 M urea to have a final concentration of 5 M urea. Carrier ampholytes, Bio-Lyte 3/5, were added to obtain a final concentration of 2% (w/v). The volume of the mixture was 50 ml. As anolyte 0.1 M $H_3PO_4$, and as catholyte 0.1 M NaOH, were used. To bring the sample to the running temperature (4°C), the cell was rotated for 15 minutes before applying electric current. The running conditions were typical as follows: constant power 12 watts; 466 volts and 26 milliamps. After 4 hours when the run was completed, the voltge reached 698 volts and the current was 17 milliamps. At the completion of the run, the pH gradient was determined on the 20 harvested fractions (2 ml each), diluted 1:10 with freshly boiled double-distilled water. For refractionation the fractions showing inhibitory activity, having a pH of 4.03 to 4.21, were pooled and diluted With 10 M urea to a final concentration of 5 M urea. The run was performed as above, without adding additional carrier ampholytes. The beginning running conditions were as follows: constant power 12 watts; 1087 volts and 12 milliamps. At the end of the run, the voltage reached 1828 volts and the current was 7 milliamps.

To remove the carrier ampholytes, the harvested fractions were dialyzed against 1 M NaCl, followed by dialysis against RPMI 1640. The purity of the fractions showing activity in the comitogenicity assay was determined by analytical isolectric focusing in 0.5 mm thin-layer polyacrylamide gels, using the LKB 2117 Multiphor System and by SDS-PAGE using the Mini-Protein 11 slab cell of Bio-Rad.

### 6.2. BIOCHEMICAL CHARACTERIZATION

The Il-1 inhibitor was compared directly to the inhibitor from M20 cells by the co-mitogenicity assay. Both inhibitors were purified by ion-exchange chromatography (gradient 0.0-0.4 M NaCl) under the same conditions and tested for their inhibitory activity at various dilutions.

Separately, fractions of the IL-1 inhibitor with peak inhibitory activity from an ion-exchange chromatography were analyzed by gel-filtration chromatography on a Sephadex G-200 Superfine column (Pharmacia). The samples were dialyzed, lyophilized, dissolved in a small volume of buffer and 2.0 ml was run. As before, each new fraction was assayed for its ability to inhibit the co-mitogenic effect of IL-1 on mouse thymocytes.

Peak fractions of IL-1 inhibitor activity from both the ion-exchange and the gel-filtration chromatography (i.e. before and after the G-200 column) were run in adjacent lanes on a SDS-polyacrylimide electrophoretic gel with molecular weight markers.

IL-1 receptor-binding assays were performed as follows. Mouse thymocytes were plated into 24-well plates and cultured until confluent. The cells were washed once with binding buffer (Dulbecco's modified Eagle's medium with 0.1% bovine serum albumin (BSA), 10 mM HEPES and 0.1% azide and incubated for a total of 5 or 6 hours at 37 C, after which the cells were washed twice with binding buffer and solubilized with 1N NaOH. Fractions of the IL-1 inhibitor from an ion-exchange HPLC were assayed at various dilutions and under different conditions. The resulting lysate was counted in a gamma counter and the percent specific-binding was calculated. The label used was $1x10^6$ CPM of human $^{125}$(I)-IL-1, both $\alpha$ and $\beta$ (Cistron), and 10 $\mu$g/ml of human IL-1, both $\alpha$ and $\beta$ (Cistron), was used as a "competitive inhibitor.

#### 6.2.1. COMPARISON WITH M20 INHIBITOR

As can be seen by the co-mitogenicity results in Table I, the IL-I inhibitor was more active than the M20 inhibitor (Barak et al., Eur. J. Immunol. 16:1449-1452, 1986; Barak et al., Lymphokine Res. 6(1), 1987, Abstr. N° 1133). at every dilution tested.

Table I

| COMPARISON BETWEEN M20-AND M20-2-PRODUCED IL-1 INHIBITORS BY CO-MITOGENICITY ASSAY | | | | |
|---|---|---|---|---|
| | CPM | Index of IL-1 Activity | Percent-Inhibition | |
| | | | M20 | M20-2 |
| IL-1 (1 unit) IL-1 (2 units) | 650<br>4200<br>9500 | <br>6.46<br>14.62 | | |
| IL-1 (2 units) inhibitor<br>diluted 1:2<br>diluted 1:4<br>diluted 1:8 | 2280<br>3990<br>5700<br>6840 | | 76<br>58<br>40<br>28 | 88<br>76<br>58<br>39 |
| inhibitor alone<br>diluted 1:2 | 635<br>643 | | 0<br>0 | 0<br>0 |

Both the M20- and M20-2-derived inhibitors were used at the same degree of purification (peaks from a DEAE ion-exchange column) at the same concentration upon 2 units of Human Recombinant IL-1.

Percent-Inhibition is defined as the CPM with IL-1 plus the inhibitor, divided by the CPM with IL-1 alone. Index of Activity is defined as the CPM with IL-1 divided by the background CPM without any IL-1.

As shown, the IL-1 inhibitor had no effect on the cells in the absence of IL-1, thus indicating its specificity.

6.2.2. GEL-FILTRATION CHROMATOGRAPHY

The fractions with peak IL-1 inhibitory activity from an ion-exchange chromatography were then characterized by gel-filtration chromatography@ fractions from which were assayed for inhibition of the co-mitogenic effect of IL-1 on mouse thymocytes.

6.2.3. SDS-PAGE

The fractions of IL-1 inhibitor, from both the ion-exchange and the subsequent gel-filtration chromatography were analyzed by SDS-PAGE (Fig. 6).

6.2.4. IN VITRO BINDING ASSAYS

IL-1 inhibitor from a previous ion-exchange HPLC (0.0-0.4 M gradient) was analyzed by three types of IL-1 receptor binding assays. In the first, fractions from the HPLC (9, I5, 10-16 XC 15, and 43-45), in either 1:50 or 1:100 dilutions were pre-incubated with mouse thymocytes for 1 hour at 37° C. As a comparison, an identical set of cells was treated with the same dilutions of just binding buffer (i.e. no inhibitor yet), under the same conditions. Next, both the pre-incubated and the other part of the assay were exposed to $10^6$ CPM of of $^{125}$(I)-IL-1, $\alpha$ or $\beta$, for 4 hours at 37° C, and the previously buffer-only segment was also simultaneously incubated with the same fractions, at the same dilutions, of the IL-1 inhibitor. Both controls were treated in a similar manner, with either IL-1, $\alpha$ or $\beta$, 10 u/ml, in one control, or the just the label, $10^6$ CPM of $^{125}$(I)-IL-1, $\alpha$ or $\beta$, in the other controls replacing the fractions of inhibitor in both the pre-incubated and simultaneous segments of the assay. The percent-specific binding was calculated as:

$$100 \times \left(\frac{\text{CPM with inhibitor - CPM with IL-1}}{\text{CPM label only - CPM with IL-1}}\right)$$

The results are shown in Table II.

TABLE II

IN VITRO BINDING ASSAY FOR IL-1 INHIBITOR

| SAMPLE | Dilution | Preincubation | IL-1 | CPM | Percent-binding |
|---|---|---|---|---|---|
| | 1:50 | + | α | 12976 | 111 |
| | " | + | β | 2673 | 91 |
| 9 | " | - | α | 9036 | 92 |
| | " | - | β | 3126 | 97 |
| | 1:100 | + | α | 13490 | 122 |
| | " | + | β | 2659 | 90 |
| | " | - | α | 9814 | 104 |
| | " | - | β | 3328 | 127 |
| | 1:50 | + | α | 12945 | 111 |
| | " | + | β | 2808 | 102 |
| 15 | " | - | α | 8760 | 88 |
| | " | + | β | 3201 | 108 |
| | 1:100 | + | α | 12368 | 104 |
| 15 | " | - | β | 2878 | 107 |
| | " | - | α | 8026 | 77 |
| | " | - | β | 3015 | 81 |
| | 1:50 | + | α | 12088 | 100 |
| | " | + | β | 2727 | 95 |
| 43-45 | " | - | α | 12211 | 102 |
| | " | - | β | 3102 | 94 |
| | 1:100 | - | α | 12186 | 102 |
| | " | + | β | 2656 | 90 |
| 43-45 | " | + | α | ND | ND |
| | " | - | β | 3152 | 101 |
| | 1:50 | + | α | 4035 | 4 |
| | " | + | β | 1999 | 39 |
| 10-16 | " | - | α | 3944 | 17 |
| xc 15 | " | - | β | 2788 | 48 |
| | 1:100 | + | α | 6823 | 38 |
| | " | + | β | 2368 | 68 |
| 10-16 | " | - | α | 5530 | 41 |
| xc 15 | " | - | β | ND | ND |
| | 10 ug per ml | + | α | 3675 | 0 |
| | " | + | β | 1490 | 0 |
| IL-1 | " | - | α | 2762 | 0 |
| | " | - | β | 2460 | 0 |
| | - | + | α | 12050 | 100 |
| | - | + | β | 2787 | 100 |
| | - | - | α | 9570 | 100 |
| | - | - | β | 3144 | 100 |

"ND" = not done

The results demonstrate that of the fractions tested, only 10-16 XC 15 contained any IL-1 receptor-binding inhibitor. This inhibitor blocked the binding of both IL-1 α and β, and did not require pre-incubation with the cells prior to the addition of $^{125}$(I)-IL-1 to demonstrate this inhibition, although the effect is somewhat increased by pre-incubation.

For the two remaining assays, only $^{125}$(I)-IL-1 α and IL-1 α were used and the only inhibitor fraction tested was 10-16 XC 15.

In the next type of receptor-binding assay, multiple dilutions of the inhibitor were tested for inhibitory activity after different treatments, in order to observe the inhibitor's retention of binding-capability. The protocol was the same as that for the simultaneous segment of the previous assay. The percent inhibition of binding is:

100 - (percent-specific binding).

The results are shown in Table III.

Table III

| IN VITRO BINDING OF IL-1 INHIBITOR AFTER EXPOSURE TO HEAT | | | |
|---|---|---|---|
| Dilution | Treatment | CPM Bound | Percent-Inhibition |
| 1:10 | none | 1597 | 99 |
| 1:20 | " | 1620 | 98 |
| 1:40 | " | 2020 | 90 |
| 1:80 | " | 2613 | 77 |
| 1:160 | " | 3015 | 68 |
| 1:320 | " | 3307 | 61 |
| 1:10 | 100°, 2′ | 5361 | 16 |
| 1:20 | " | 5557 | 12 |
| 1:40 | " | 5601 | 11 |
| 1:80 | " | 5778 | 7 |
| 1:160 | " | 6037 | 2 |
| 1:320 | " | 6108 | 0 |
| 1:10 | 56°, 20′ | 1594 | 99 |
| 1:20 | " | 1938 | 91 |
| 1:40 | " | 2407 | 81 |
| 1:80 | " | 3007 | 68 |
| 1:160 | " | 3397 | 59 |
| 1:320 | " | 4145 | 43 |
| IL-1 (10 ug per ml) | | 1542 | 100 |
| No inhibitor | | 6112 | 0 |

As can also be seen Figure 7, the inhibitor's activity, relative to untreated inhibitor, was destroyed if it was incubated for 2 minutes at 100°C, while incubation for 20 minutes at 56°C had little, if any, effect on the inhibitory activity, implying that the substance responsible for the inhibitor's activity is proteinaceous.

The last type of receptor-binding assay tested the degree of reversibility of the inhibitor's binding. The protocol was the same as that for the pre-incubated segment of the first assay, except that the inhibitor, at multiple dilutions, was pre-incubated for 2 hours (instead of only 1 hour) at 37°C, after which the excess, unbound IL-1 inhibitor was removed from half of the cells at each dilution. The results are shown in Table IV:

TABLE IV

| REVERSABILITY OF IN VITRO BINDING OF IL-1 INHIBITOR | | | |
|---|---|---|---|
| Dilution | Inhibitor | CPM Bound | Percent-Inhibition* |
| 1:10 | + | 3185 | 95 |
| " | - | 1932 | 98 |
| 1:30 | + | 3508 | 87 |
| " | - | 1697 | 100 |
| 1:90 | + | 3753 | 81 |
| " | - | 2764 | 78 |
| 1:270 | + | 5235 | 46 |
| " | - | 3483 | 60 |
| 1:810 | + | 5764 | 31 |
| " | - | 4122 | 45 |
| 1:2430 | + | 5476 | 38 |
| " | - | 5372 | 14 |
| 1:7290 | + | 6561 | 11 |
| " | - | 5630 | 8 |
| 1:21870 | + | 7599 | 0 |
| " | - | 5046 | 22 |
| 1:65610 | + | 7662 | 0 |
| " | - | 5499 | 12 |
| 10 ug per ml of IL-1 | + | 2982 | 100 |
| " | - | 1848 | 100 |
| no inhibitor | + | 7020 | 0 |
| " | - | 6055 | 0 |

* Relative to identical treatment of cells with 10 ug per ml of IL-1.

The results, as also shown in Figure 8, suggest that the IL-1 inhibitor disassociates from the IL-1 receptor as readily as IL-1 itself does, because it makes no difference in the degree of competition whether the inhibitor is removed or maintained in competition with the labeled IL-1. Thus, IL-1 and its inhibitor appear to contain a similar affinity for the IL-1 receptor.

6.3. ACTIVITY IN VIVO

IL-1 inhibitor, from the peak of inhibitory activity from an ion-exchange chromatography of culture media from M20-2 cells, was shown to have anti-inflammatory activity by three art-recognized tests.

6.3.1. INHIBITION OF FEVER INDUCTION

As described above, the IL-1 inhibitor ability to inhibit the induction of fever was tested in mice, with the results presented as "mean body temperature ± standard error (SE)" for each group of two mice injected with the same compound. The differences in temperature within each group over time are given as $\Delta T$ values.

Body temperature was measured after increasing elapsed times following the concomitant injection of 150 units of IL-1 and 1 or 2 units of the inhibitor, with control groups injected with PBS or human IL-1 alone. The results are shown in Table V.

TABLE V

INHIBITION OF IL-1 INDUCED FEVER BY CONCOMITANT INJECTION OF IL-1 INHIBITOR

| | | | Time after Injection (hr) | | | | | | | | |
|---|---|---|---|---|---|---|---|---|---|---|---|
| | | Amount of IL-1 inhibitor per mouse | 0 | 1/2 | | 1 | | 1 1/2 | | 2 | |
| Exp. No. | Injection PBS | | Body temp. ND | Δ T* ND | % Inh. ND | Δ T ND | % Inh. ND | Δ T ND | % Inh. ND | Δ T ND | % Inh. ND |
| 1 | IL-1 | - | 37.7 | 0.22±0.14 | - | 0.46±0.14 | - | 0.24±0.09 | - | 0.02±0.11 | - |
| | plus inhibitor | 2 units | 37.7 | -0.12±0.12 | 100 | -0.72±0.18 | 100 | -0.22±0.16 | 100 | 0.02±0.11 | 100 |
| 2 | PBS | - | 36.87 | 0.1 | - | 0.13±0.09 | - | 0.13±0.05 | - | 0.06±0.07 | - |
| | IL-1 | - | 36.57 | 0.43±0.04 | - | 1.3 ±0.11 | - | 0.7 ±0.10 | - | 0.22±0.05 | - |
| | plus inhibitor | 2 units | 36.72 | -0.2 ±0.03 | 100 | 0.15±0.05 | 100 | 0.08±0.06 | 100 | 0.13±0.06 | 100 |
| 3 | PBS | - | 36.67 | 0 | - | -0.1 ±0.06 | - | -0.05±0.04 | - | 0± | - |
| | IL-1 | - | 36.52 | 0.52±0.02 | - | 1.52±0.11 | - | 0.6 ±0.06 | - | 0.22±0.05 | - |
| | plus inhibitor | 2 units | 36.67 | 0.13±0.02 | 100 | -0.15±0.06 | 100 | 0.03±0.06 | 95 | 0.02±0.06 | 89 |
| 4 | PBS | - | 36.90 | 0.12±0.04 | - | 0.12±0.03 | - | 0.07±0.02 | - | 0.07±0.02 | - |
| | IL-1 | - | 36.70 | 0.45±0.06 | - | 1.47±0.09 | - | 0.7 ±0.11 | - | 0.27±0.1 | - |
| | plus inhibitor | 1 unit | 36.47 | 0.2 ±0.07 | 55 | 0.72±0.08 | 50 | 0.4 ±0.08 | 43 | 0.15±0.02 | 45 |

* Δ T : change in body temperature

In all four experiments a peak of increase in body temperature was observed 1 hour after injection with IL-1 alone, while a complete inhibition of temperature elevation was observed at all four time points, in all four experiments, when the IL-1 inhibitor was concomitantly injected.

Next, the effect of varying amounts of the inhibitor on IL-1 fever induction was tested by measuring the body temperature at different time points after the concomitant injection of IL-I and the inhibitor (Fig. 9). A gradual increase in the inhibition of fever with increasing amounts of inhibitor, independent of time, was observed, with a range of about 50% inhibition obtained with 1 unit of inhibitor.

Finally, the effect of the time interval between the injection of 150 units of IL-1 and 2 units of the inhibitor on body temperature was tested at 1.0 and 1.5 hours after the IL-1 injection (Fig. 10). The results indicate that fever inhibition was exhibited when the inhibitor was injected from 2 to 16 hours before the injection of IL-1, with a decrease in inhibition when the inhibitor was injected up to 24 hours before. Thus, the inhibitor reversed the fever induced by IL-1 even if injected 24 hours before the IL-1.

### 6.3.2. INHIBITION OF LEUKOCYTOSIS INDUCTION

The ability of the IL-1 inhibitor to inhibit the induction by IL-1 of leukocytosis in mice was assayed by the index of leukocytosis, defined as the mean (±SE) leukocyte counts in a group injected with human IL-1 alone or in conjunction with the IL-1 inhibitor, divided by the mean (±SE) counts in a group injected with PBS only (the standard error, for all data was between 0.0350 and 0.0127).

In general, the peak increase in leukocyte counts was observed 2 to 3 hours after the injection of I50 to 200 units of IL-1 per mouse (Fig. 11). The concomitant injection of 2 units of inhibitor always caused a reduction of this IL-1 effect, with maximal inhibition obtained 2 to 3 hours after the injection.

As found for fever induction, inhibition of leukocytosis was also observed when IL-1 and the inhibitor were injected separately, at varying time intervals (Fig. 12). The inhibitor reduced the leukocytosis induced by IL-1 even if injected 2 to 24 hours before the IL-1, but the peak inhibitory effect, at 4 hours, was decreased if the time interval was increased.

### 6.3.3. INHIBITION OF LOCAL LYMPH NODE-ENLARGEMENT INDUCTION

To assay the ability of the IL-1 inhibitor to affect the induction, by various agents, of local lymph node enlargement, the index of enlargement was determined by the mean (±SE) weight of the right lymph nodes of the mouse (injected with IL-1 alone or in conjunction with the inhibitor) divided by the mean (±SE) weight of the left lymph nodes, injected with just PBS.

TABLE VI

| EFFECT OF IL-1 INHIBITOR ON LOCAL LYMPH NODE ENLARGEMENT | | | | |
|---|---|---|---|---|
| Inducer | Amount Per Mouse | IL-1 Inhibitor (2 units) | Index of Enlargement | Percent Inhibition |
| Con A | 12.5 µg | - | 3.73±0.07 | - |
| | 12.5 µg | + | 1.17±0.04 | 69 |
| LPS | 50 µg | - | 4.85±0.27 | - |
| | 50 µg | + | 1.19±0.07 | 75 |
| Mouse IL-1 | 125 units | - | 4.09±0.11 | - |
| | 125 units | + | 1.26±0.11 | 70 |
| Human IL-1 | 100 units | - | 3.76±0.06 | - |
| | 100 units | + | 0.89±0.09 | 77 |

The results in Table VI represent the optimal lymph node enlargement induced by mitogens such as Con A and LPS as well as both human and mouse IL-1. In all of these cases, the concomitant injection of 2 units of the IL-1 inhibitor caused a significant reduction in the lymph node response.

The inhibitory effect on the induction by mouse IL-1 of lymph node enlargement was also tested when IL-1 and the inhibitor were injected separately, at varying time intervals. The results are shown in Table VII.

TABLE VII

| EFFECT OF IL-1 INHIBITOR ON LOCAL LYMPH NODE ENLARGEMENT | | | | | |
|---|---|---|---|---|---|
| Time Interval Between IL-1 and Inhibitor Injection | Lymph Node Weight (mg) | | Index of Enlargement | | Percent Inhibition |
| | Right | Left | | | |
| IL-1 (no inhibitor) | 0.76<br>1.12<br>0.94<br>1.07 | 4.28<br>3.94<br>3.82<br>3.88 | 5.63)<br>3.51)<br>4.06)<br>3.62) | 4.20±0.42 | - |
| 4 days before | 0.86<br>1.08 | 3.53<br>3.78 | 4.10)<br>3.50) | 3.80±0.21 | 10 |
| 3 days before | 1.30<br>1.24 | 3.42<br>3.88 | 2.63)<br>3.12) | 2.87±0.17 | 31 |
| 2 days before | 1.94<br>1.26 | 3.25<br>3.78 | 1.68)<br>1.96) | 1.82±0.1 | 56 |
| 1 day before | 0.85<br>1.28 | 1.36<br>1.70 | 1.32)<br>1.32) | 1.46±0.1 | 65 |
| 2 hours before | 0.96<br>1.42<br>1.02 | 1.56<br>3.98<br>2.32 | 1.62)<br>2.80)<br>2.27 | 2.23±0.27 | 47 |
| 2 hours after | 1.05<br>1.38<br>1.36 | 2.96<br>2.56<br>2.84 | 2.81)<br>1.85)<br>2.08) | 2.24±0.23 | 47 |
| 1 day after | 0.92<br>0.78 | 2.98<br>2.67 | 3.24)<br>3.42) | 3.33±0.07 | 21 |

Injection of 1 unit of the inhibitor of 2 hours, 1 day or 2 days before 125 units of IL-1 still caused significant inhibition. The inhibition was lower if the time between was increased to 3 days and only marginal by 4 days. A significant inhibition was also observed if the inhibitor was injected 2 hours after the IL-1, but this decreased when the interval was increased to 1 day.

7. DEPOSIT OF MICROOGANISMS

The following M20-2 cell line has been deposited on April 4, 1990 with the American Type Culture Collection, Rockville, Maryland, and has been assigned the listed accession number:

| Cell Line | Accession Number |
|---|---|
| M20-2 | ATCC CRL 10409 |

## Claims

1. A 52±4 kD molecular weight protein, isolatable from a supernatant of the cell line M20-2, said protein being characterized by a pI of 4.1 ± 0.2 and an ability to inhibit or reduce an IL-1 mediated inflammatory response.

2. The protein of Claim 1 which is isolatable from fractions of the supernatant resulting from the first step of purification by ion-exchange chromatography.

**3.** The protein of Claim 2 which is further purified by ion-exchange, high-performance liquid chromatography.

**4.** The protein of Claim 1 which is isolatable from the supernatant by ion-exchange, high-performance liquid chromatography.

**5.** The protein of Claim 1 which is isolatable from the supernatant by isoelectric focusing.

**6.** A therapeutic composition comprising an anti-inflammatory-effective amount of the protein of Claim 1, 2, 3, 4 or 5, in combination with a pharmaceutically acceptable carrier.

**7.** A pharmaceutical composition for inhibiting or reducing an inflammatory response which comprises administering to an individual in need of such treatment an effective amount of the protein of Claim 1, 2, 3, 4 or 5.

**8.** The composition of Claim 7 wherein the amount is about 0.0001-1.0 µg per kg of body weight.

**9.** A substantially pure protein free of major contaminants as shown by the presence of one band in SDS-PAGE, the protein having IL-1 inhibitory activity, a molecular weight of 52 ± 4 kD, an isoelectric point of 4.1 ± 0.2, isolatable from a supernatant of the cell line M20-2, and active fragments thereof.

**Patentansprüche**

**1.** Protein mit einem Molekulargewicht von 52 ± 4 kD, isolierbar aus einer überstehenden Flüssigkeit der Zellinie M20-2, wobei das Protein durch einen PI von 4,1 ± 0,2 und eine Fähigkeit, IL-1 vermittelte Entzündungsreaktionen zu hemmen oder zu reduzieren, gekennzeichnet ist.

**2.** Protein nach Anspruch 1, das isolierbar ist aus Fraktionen der überstehenden Flüssigkeit, die aus der ersten Stufe der Reinigung durch Ionenaustauch-Chromatographie resultiert.

**3.** Protein nach Anspruch 2, das weiter gereinigt wird durch Ionenaustausch, Hochleistungs-Flüssigchromatographie.

**4.** Protein nach Anspruch 1, welches isolierbar ist aus der überstehenden Flüssigkeit durch Ionenaustausch, Hochleistungs-Flüssigchromatographie.

**5.** Protein nach Anspruch 1, welches isolierbar ist aus der überstehenden Flüssigkeit durch isoelektrische Fokusierung.

**6.** Therapeutische Zusammensetzung, umfassend eine anti-inflammatorisch wirksame Menge des Proteins nach Anspruch 1, 2, 3, 4 oder 5, zusammen mit einem pharmazeutisch brauchbaren Träger.

**7.** Pharmazeutische Zusammensetzung zur Hemmung oder Reduzierung einer Entzündungsreaktion, welche umfaßt, einer Person, die solch eine Behandlung benötigt, eine wirksame Menge des Proteins nach Anspruch 1, 2, 3, 4 oder 5 zu verabreichen.

**8.** Zusammensetzung nach Anspruch 7, worin die Menge ungefähr 0,0001-1,0 µg pro kg Körpergewicht ist.

**9.** Ein im wesentlichen reines Protein, frei von größeren Verschmutzungen, gezeigt durch das Vorhandensein von einer Bande in der SDS-PAGE, wobei das Protein IL-1 hemmende Aktivität, ein Molekulargewicht von 52 ± 4 kD und einen isoelektrischen Punkt bei 4,1 ± 0,2 besitzt und aus einer überstehenden Flüssigkeit der Zellinie M20-2 isolierbar ist und aktive Fragmente davon.

**Revendications**

**1.** Protéine d'un poids moléculaire de 52 ± 4 kD, isolable à partir d'un surnageant de la lignée cellulaire M20-2, ladite protéine étant caractérisée par un pI de 4,1 ± 0,2 et une aptitude à inhiber ou à réduire une réponse inflammatoire passant par la médiation d'IL-1.

**2.** Protéine de la revendication 1 qui est isolable à partir de fractions du surnageant résultant de la première étape de purification par chromatographie d'échange d'ions.

**3.** Protéine de la revendication 2 qui est encore purifiée par une chromatographie d'échange d'ions en phase liquide à haute performance.

**4.** Protéine de la revendication 1 qui est isolable à partir du surnageant par une chromatographie d'échange d'ions en phase liquide à haute performance.

**5.** Protéine de la revendication 1 qui est isolable à partir du surnageant par focalisation isoélectrique.

**6.** Composition thérapeutique comprenant une quantité efficace comme anti-inflammatoire de la protéine de la revendication 1, 2, 3, 4 ou 5, en combinaison avec un support pharmaceutiquement acceptable.

**7.** Composition pharmaceutique pour inhiber ou réduire une réponse inflammatoire dans laquelle on administre à un individu ayant besoin d'un tel traitement une quantité efficace de la protéine de la revendication 1, 2, 3, 4 ou 5.

**8.** Composition de la revendication 7 dans laquelle la quantité est d'environ 0,0001-1,0 $\mu$g par kg de poids corporel.

**9.** Protéine sensiblement pure dépourvue d'impuretés majeures comme le montre la présence d'une bande à l'électrophorèse sur gel de dodécyl-sulfate de sodiumpolyacrylamide (SDS-PAGE), la protéine ayant une activité inhibitrice d'IL-1, un poids moléculaire de 52 ± 4 kD, un point isoélectrique de 4,1 ± 0,2, isolable à partir d'un surnageant de la lignée cellulaire M20-2, et ses fragments actifs.

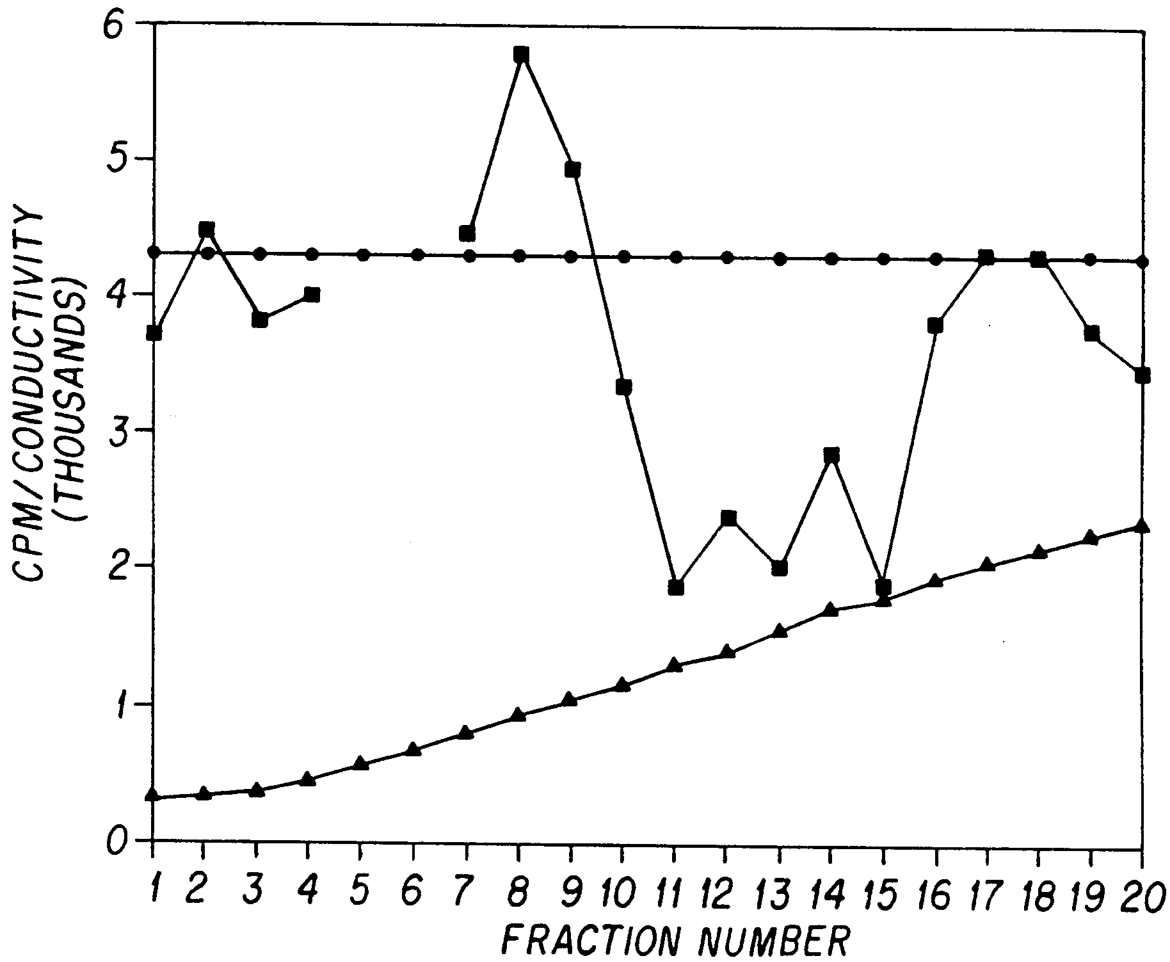
6
5
4
3
2
1
0
CPM/CONDUCTIVITY
(THOUSANDS)
1 2 3 4 5 6 7 8 9 10 11 12 13 14 15 16 17 18 19 20
FRACTION NUMBER

FIG. 1

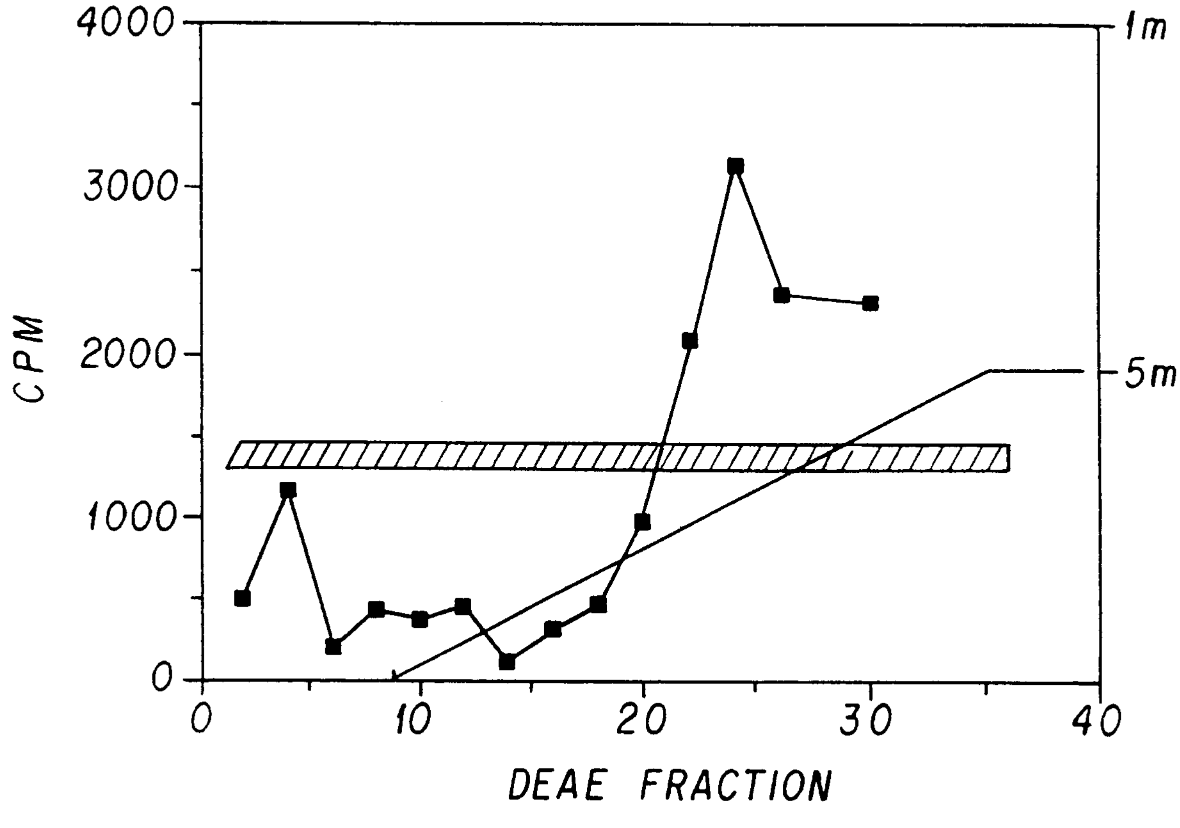


FIG.2

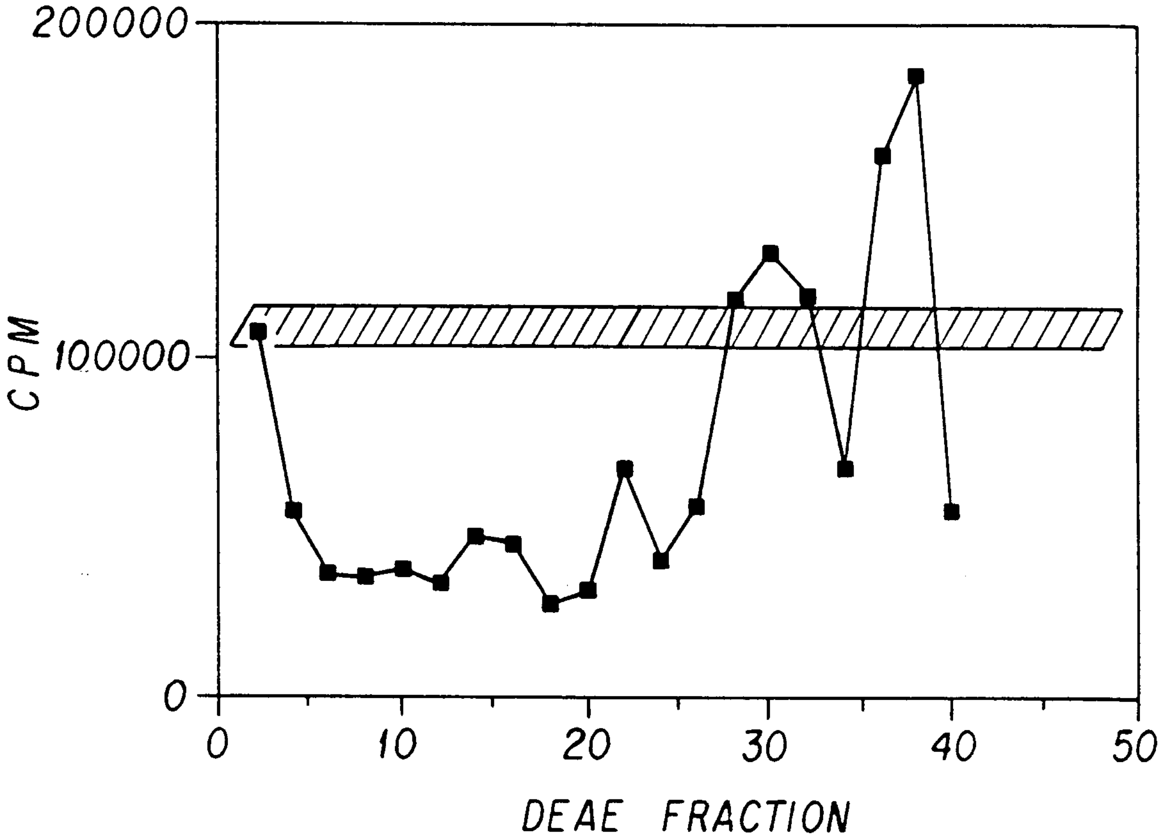


FIG. 3

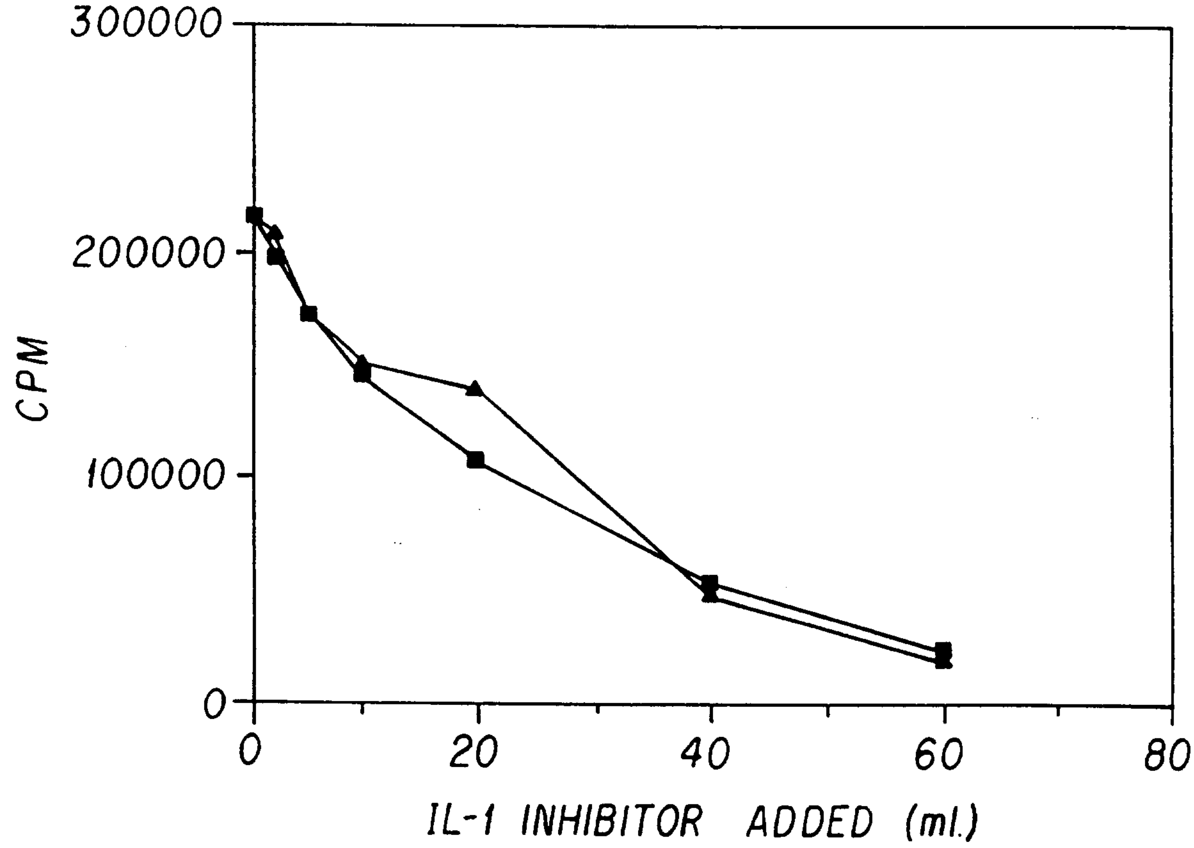
300000
200000
100000
0
CPM
0
20
40
60
80
IL-1 INHIBITOR ADDED (ml.)

FIG. 4

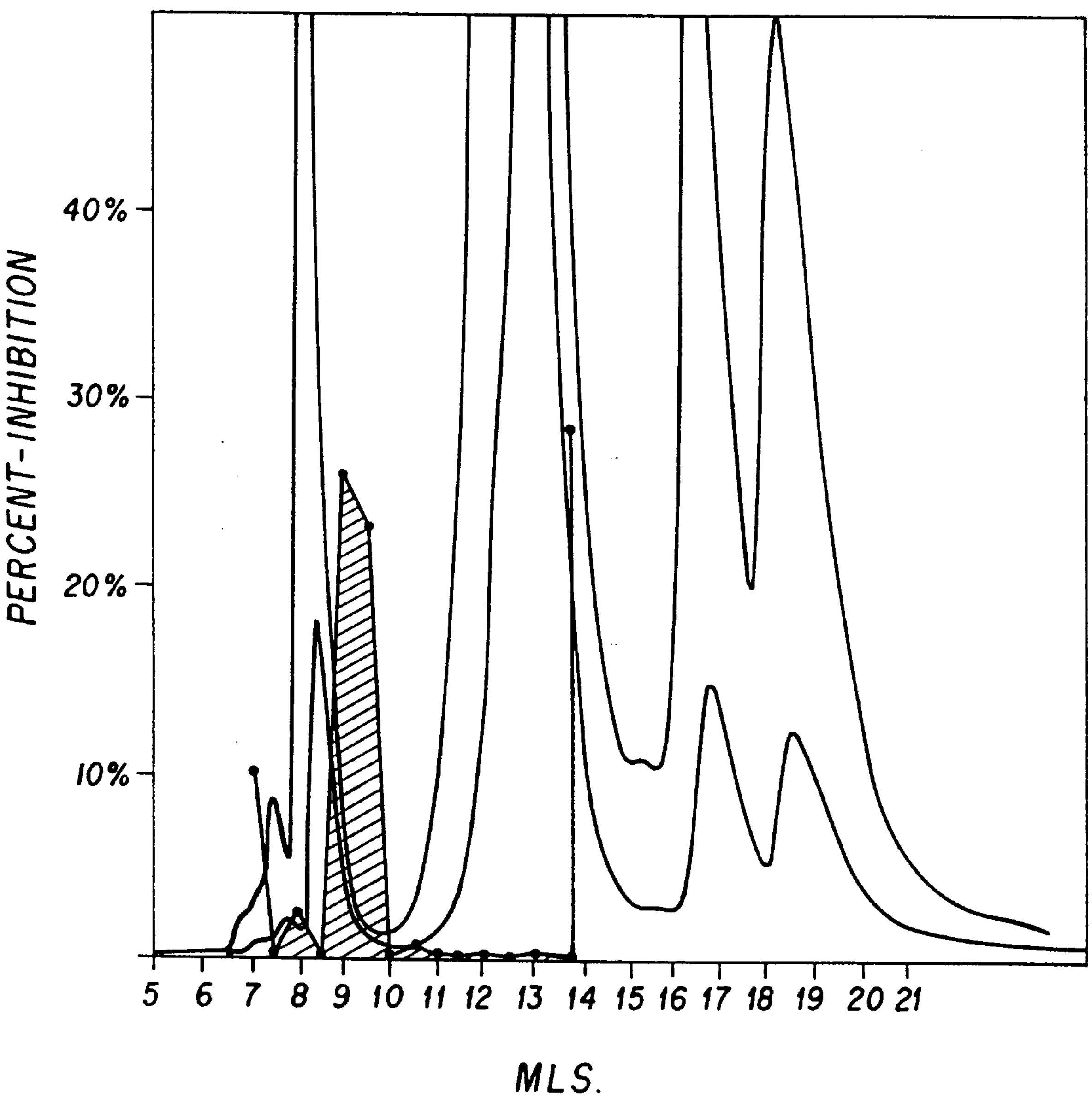
40%
30%
20%
10%
PERCENT-INHIBITION
5 6 7 8 9 10 11 12 13 14 15 16 17 18 19 20 21
MLS.

FIG. 5

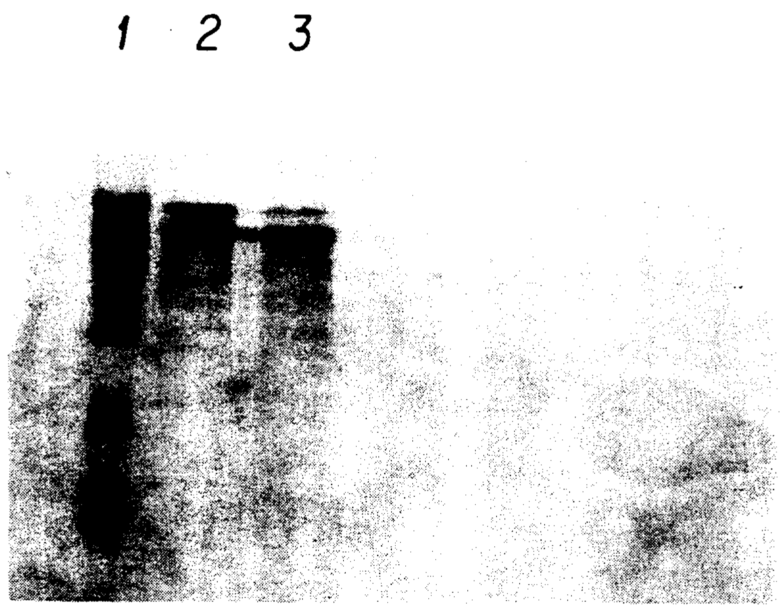


FIG. 6

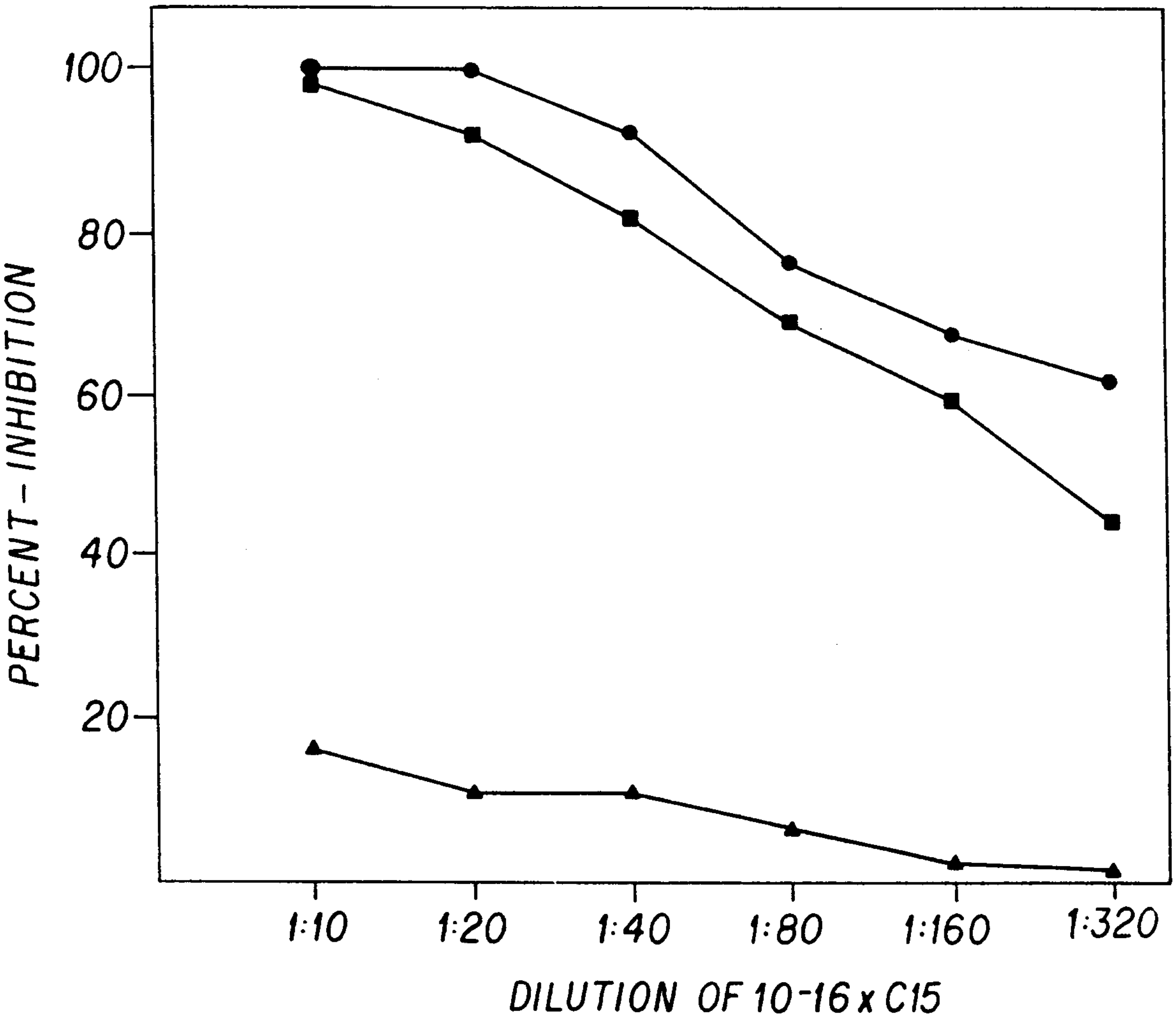
100
80
60
40
20
PERCENT - INHIBITION
1:10
1:20
1:40
1:80
1:160
1:320
DILUTION OF 10-16 x C15

FIG. 7

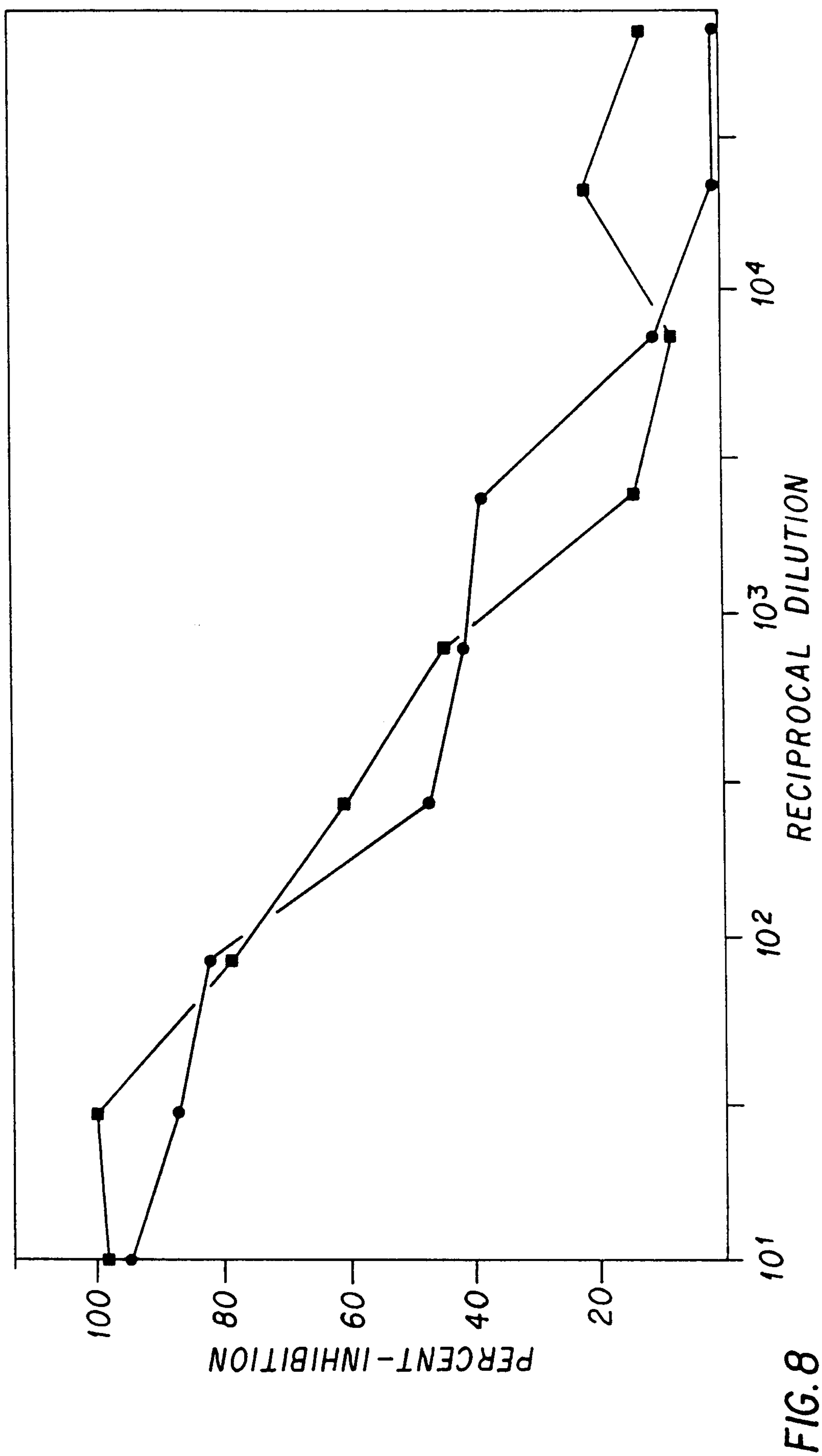
PERCENT-INHIBITION
100
80
60
40
20
RECIPROCAL DILUTION
$10^1$
$10^2$
$10^3$
$10^4$

FIG. 8

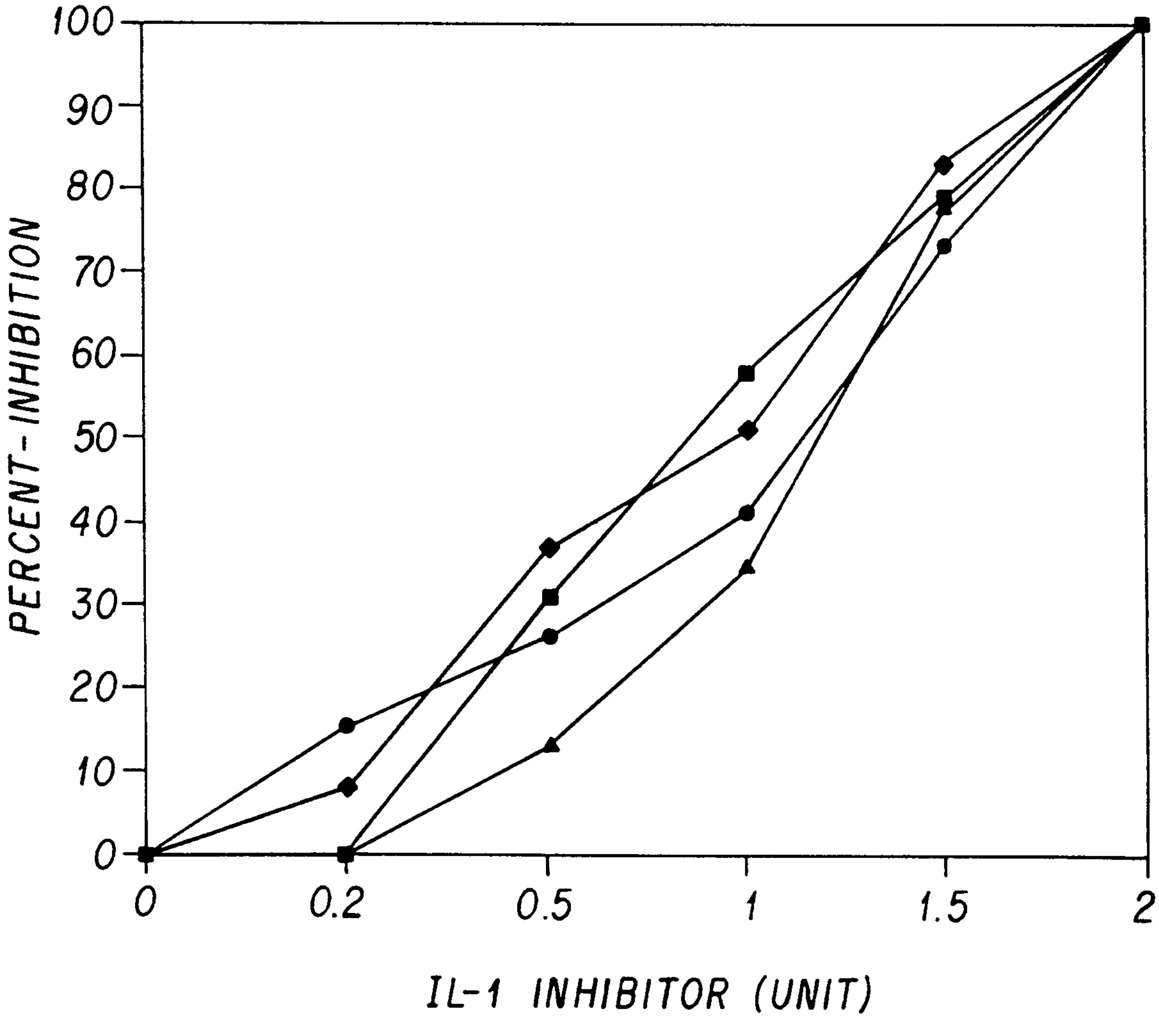
100
90
80
70
60
50
40
30
20
10
0
PERCENT-INHIBITION
0
0.2
0.5
1
1.5
2
IL-1 INHIBITOR (UNIT)

FIG. 9

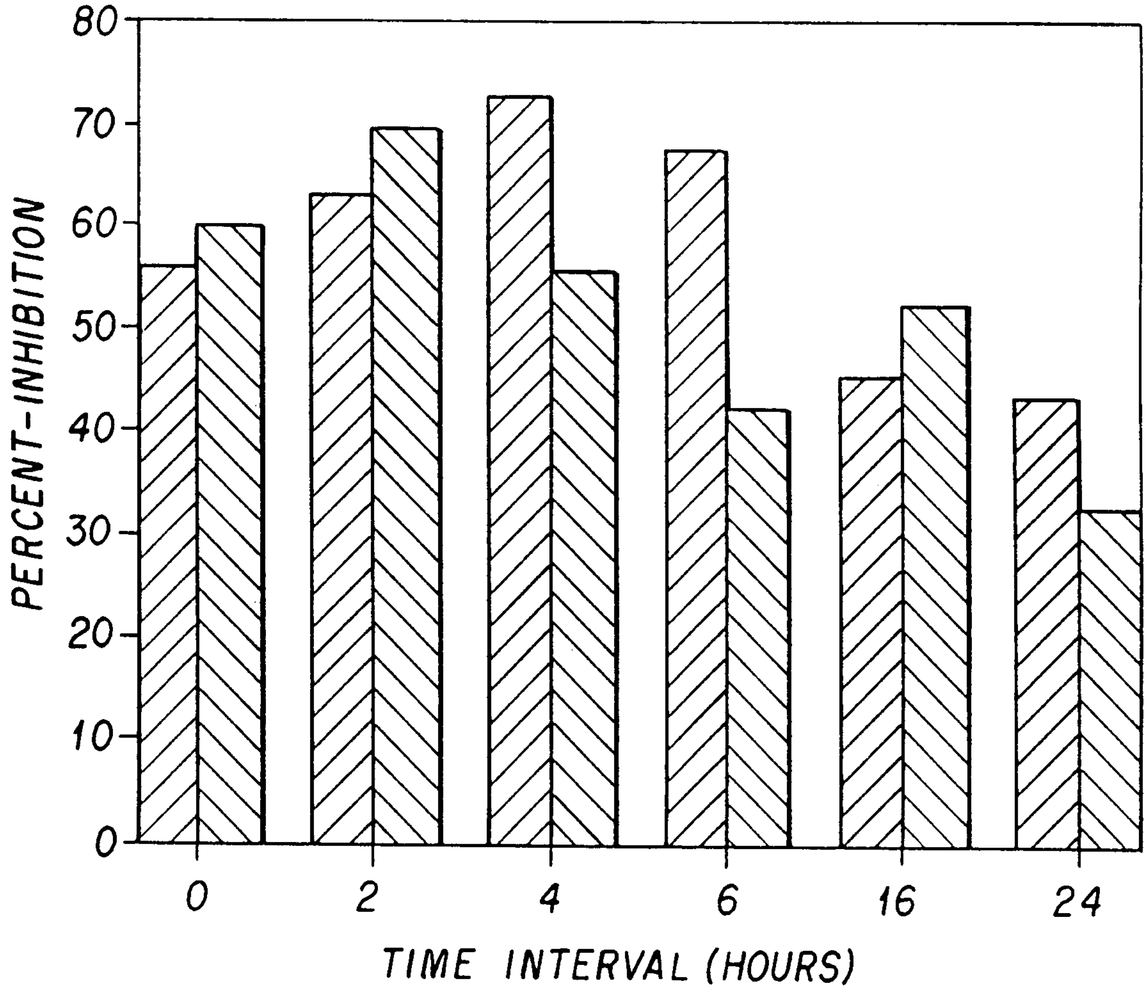
80
70
60
50
40
30
20
10
0
PERCENT-INHIBITION
0
2
4
6
16
24
TIME INTERVAL (HOURS)

FIG. 10

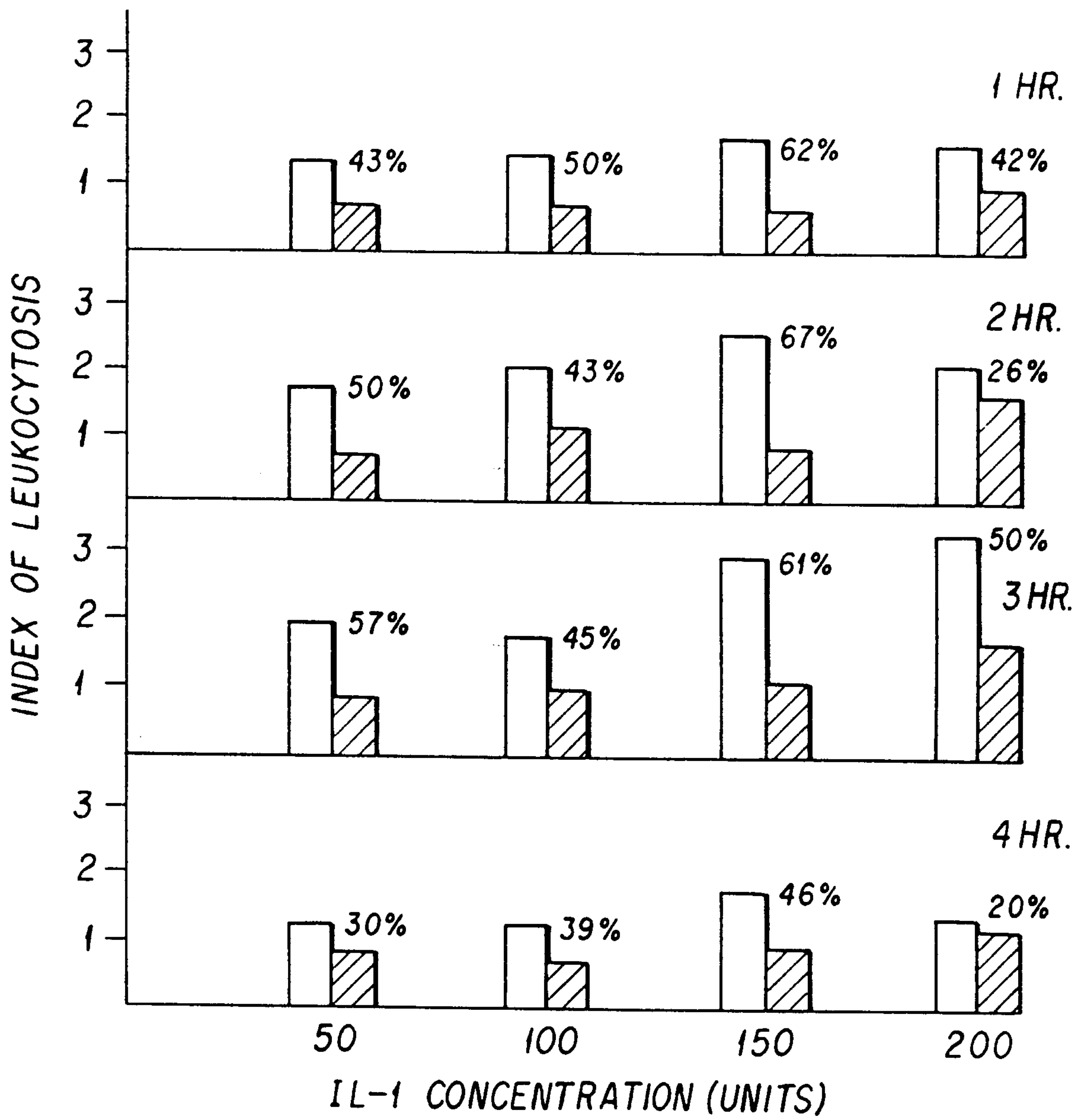
INDEX OF LEUKOCYTOSIS
3
2
1
1 HR.
43%
50%
62%
42%
2 HR.
50%
43%
67%
26%
3 HR.
57%
45%
61%
50%
4 HR.
30%
39%
46%
20%
50
100
150
200
IL-1 CONCENTRATION (UNITS)

FIG. 11

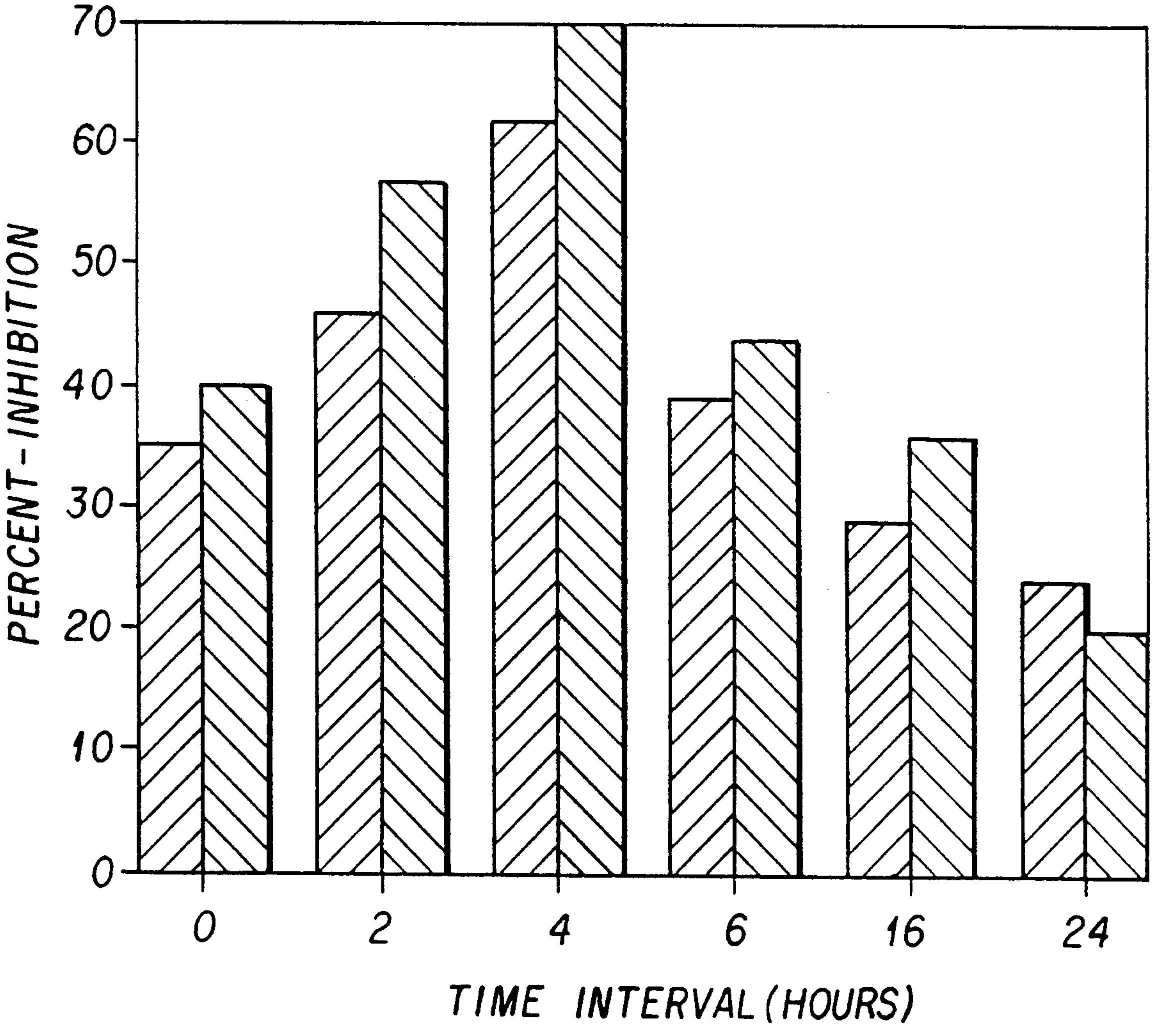
70
60
50
40
30
20
10
0
PERCENT - INHIBITION
0
2
4
6
16
24
TIME INTERVAL (HOURS)

FIG. 12